Europäisches Patentamt

European Patent Office

Office européen des brevets

(19)

(11) Publication number: **0 070 163**
A2

(12) # EUROPEAN PATENT APPLICATION

(21) Application number: 82303605.8

(22) Date of filing: 09.07.82

(51) Int. Cl.³: **D 04 H 1/54**

(30) Priority: 10.07.81 US 282326
28.05.82 US 382731

(43) Date of publication of application:
19.01.83 Bulletin 83/3

(84) Designated Contracting States:
AT DE FR GB IT NL SE

(71) Applicant: CHICOPEE
317 George Street
New Brunswick, New Jersey 08903(US)

(72) Inventor: Mays, Alfred Thomas
6, Lynnfield Drive
East Windsor New Jersey 08520(US)

(72) Inventor: Shimalla, Charles James
6 Cardinal Court
Kendall Park New Jersey 08824(US)

(74) Representative: Jones, Alan John et al,
CARPMAELS & RANSFORD 43 Bloomsbury Square
London, WC1A 2RA(GB)

(54) Nonwoven fabric composed of polyester/polyethylene conjugate fibers.

(57) There is disclosed a thermal bonded non-woven fabric composed of polyester/polyethylene conjugate fibers, said fabric having an excellent combination of low bulk density, softness, and strength. The fabric is especially useful as a facing in absorbent products.

Fig.1

EP 0 070 163 A2

# NONWOVEN FABRIC COMPOSED OF POLYESTER/POLYETHYLENE CONJUGATE FIBERS

The invention relates to a thermal bonded nonwoven fabric composed of polyester/polyethylene conjugate fibers that is particularly useful as a facing for absorbent products.

Thermal bonded nonwoven fabrics made from conjugate fibers are receiving increasing amounts of attention. Such fabrics can be produced by methods that save considerable amounts of energy because the processes for making them need not evaporate water. The present invention provides a thermal bonded nonwoven fabric having a valuable combination of low bulk density, softness, strength, and, preferably, other attributes that make it especially useful as a facing for absorbent products.

Booker et al., in U.S. Patent No. 4,211,816, describe self-bonded nonwoven fabrics produced from sheath/core conjugate fibers having a core of isotactic polypropylene and a sheath of high density polyethylene.

Ogasawara et al., in U.S. Patent No. 3,924,045, disclose conjugate fibers containing different polymers which alternate in layers in the direction of extrusion of the fiber. Polymers disclosed include polyethylene and polyester.

Smith, in U.S. Patent No. 3,316,336, discloses polyester fibers coated with a latex of polyolefin such as polyethylene.

Ejima et al., disclose thermal bonded nonwoven fabrics made from side-by-side polyethylene-polypropylene conjugate fibers.

Wincklhofer et al., in U.S. Patent No. 3,616,160, disclose conjugate fibers comprising fibrils in a matrix, wherein the polymeric constituents disclosed include polyethylene and polyester.

Beyer et al., in U.S. Patent No. 3,713,875, disclose polyester yarns or monofilaments coated with a thermoplastic material such as polyethylene.

Bryan et al., in U.S. Patent No. 3,381,074, and Breen et al., in U.S. Patent No. 3,017,686, disclose lists of polymers that can be used in making multi-component fibers, wherein polyester and polyethylene are included in said lists.

Samuelson et al., in U.S. Patent No. 4,145,473, disclose fibers having a polyethylene sheath and a conductive core.

Davies, in U.S. Patent No. 3,511,747, and Davies et al., in U.S. Patent No. 3,595,731, have general disclosures of the use of polyethylene as a potentially adhesive component in a conjugate fiber.

Yamane et al., in U.S. Patent No. 3,900,549, disclose a composite filament produced by melt spinning a mixture of polyethylene and polyester.

Breen, in U.S. Patent No. 2,931,091, discloses a conjugate fiber having a polyethylene core and a polyester sheath.

The fabric of the invention is a thermal bonded nonwoven fabric composed of polyester/polyethylene conjugate fibers. The fabric exhibits a valuable combination of strength, low bulk density, and softness. Preferably, the fabric has excellent aqueous liquid penetration and re-wet properties which make it particularly useful as a facing for absorbent products. .

In an important aspect of the invention, an absorbent product is provided which uses this fabric as a facing.

Brief Description of the Drawings

Fig. 1 is a perspective view of a sanitary napkin employing the nonwoven fabric of the invention as a facing;

Fig. 2 is a perspective view of a disposable diaper employing the nonwoven fabric of the invention as a facing;

Fig. 3 is a perspective view of the components of a test apparatus, and their relationship, for testing the liquid rewet properties of facings on absorbent products;

Fig. 4 is a top view of one portion of Fig. 3 taken along line 4-4;

Fig. 5 is a perspective view of the components and their relationship of an apparatus for further testing for the rewet properties of facings on absorbent products;

Fig. 6 is a schematic side elevation of an arrangement of apparatus suitable for making the fabric of the invention;

Fig. 7 is a photomacrograph taken at 20X of a loose aggregation of unbonded polyester/polyethylene conjugate fibers;

Fig. 8 is a photomacrograph taken at 20X of a thermal bonded nonwoven fabric made from the fibers shown in Fig. 7;

Fig. 9 is a photomacrograph taken at 20X of a loose aggregation of unbonded polypropylene/polyethylene conjugate fibers;

Fig. 10 is a photomacrograph taken at 20X of a thermal bonded nonwoven fabric made from the fibers shown in Fig. 9; and

Fig. 11 is a schematic side elevation of an apparatus for testing the compressibility of fabrics.

The thermal bonded nonwoven fabric of the invention is made from polyester/polyethylene conjugate fibers wherein at least about 50 per cent of the surface of the individual fibers is polyethylene. It is preferred to employ sheath/core fibers with the polyethylene as the sheath and the polyester as the core. Either eccentric or concentric sheath/core fibers can be employed. The fibers will usually have a denier within the range of from about 1 to about 6, and are in excess of about 1/4-inch in length, up to about 3 or 4 inches long.

Preferably, the conjugate fibers employ high density polyethylene, that is, linear polyethylene that has a density of at least about 0.94, and a Melt Index ("M.I.") by ASTM D-1238(E) (190°C., 2160 gms.) of greater than 1, preferably greater than about 10, and more preferably from about 20 to about 50. Usually the fibers will be composed of about 40 to 60 weight per cent, and preferably 45 to 55 weight per cent, polyester, the remainder being polyethylene.

A minor proportion of other fibers, preferably nonabsorbent staple fibers such as polyester fibers, can be used along with the polyester/polyethylene conjugate fibers.

The fabrics of the invention are produced by first forming a fibrous web comprising a loose array of the conjugate fibers, as by carding, air laying, or the like. It is preferred to employ a card, or a dual rotor such as is shown by Ruffo et al. in U.S. Patent No. 3,768,118, as the web forming device, although other web forming apparatus can be employed if desired. The exact weight of the fibrous web has not been found to be narrowly critical, although useful weights have been found within the range from about 0.2 to about 4.2 ounces per square yard.

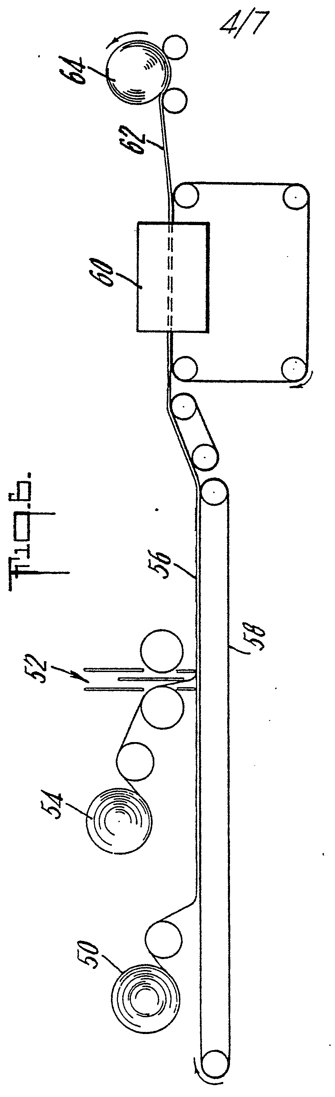

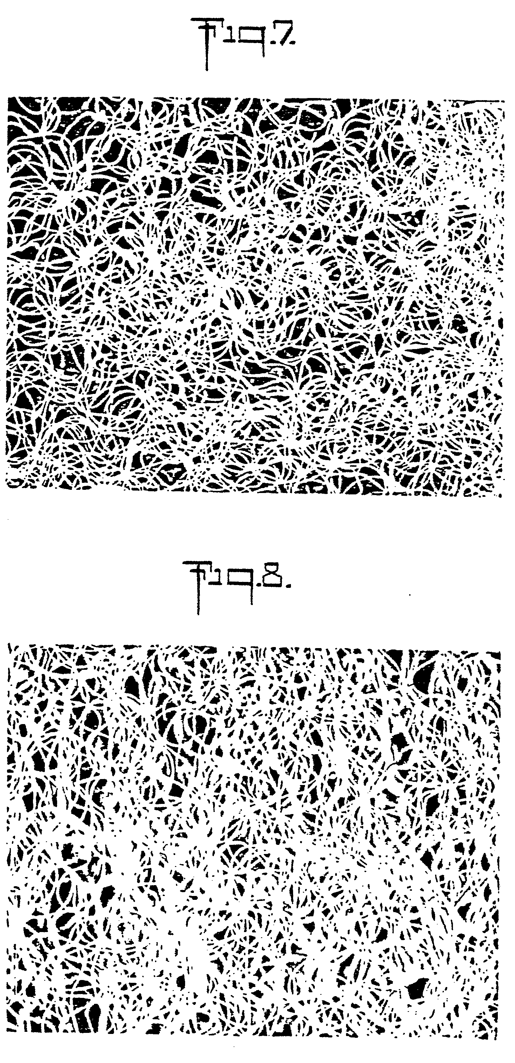

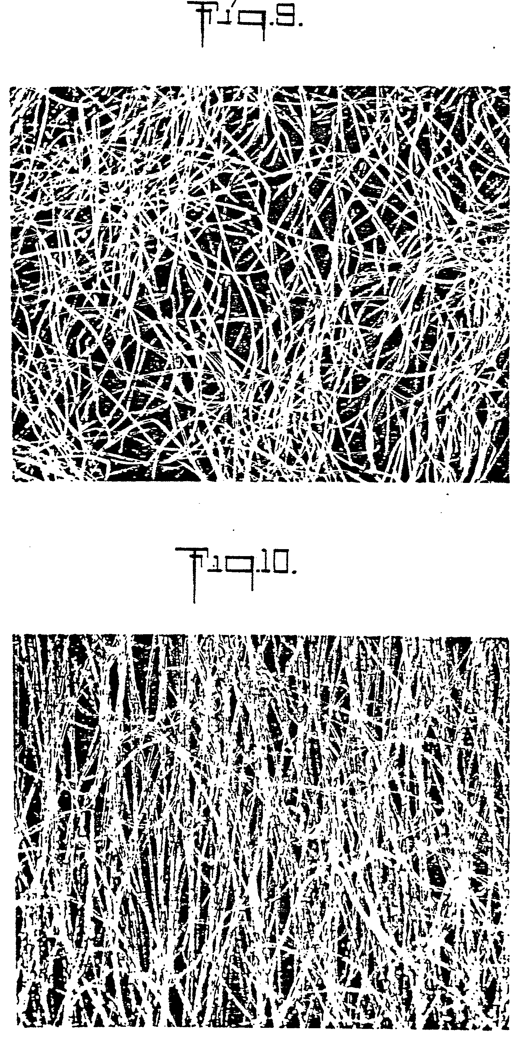

Fig. 6 shows one arrangement of apparatus that can be used to produce the fabrics of the invention. A web of loose fibers can be produced either by a card 50 or by a dual rotor 52 (which is itself preferably fed by a card 54). This web 56 comprising a loose array of polyester/polyethylene conjugate fibers is laid on an endless belt 58, and is conveyed to heating means such as a forced air oven 60. In the oven 60, the web is subjected to elevated temperature to fuse the polyethylene component of the conjugate fibers and form bonds at points of fiber-to-fiber contact. The web is thermal bonded under conditions of zero pressure, or very light pressure, so that the web is not significantly crushed or compacted during the thermal bonding step. The exact temperatures employed in the thermal bonding will vary, depending upon the weight and bulk density of the web, and upon the dwell time employed in the heated zone. For instance, bonding temperatures within the range from about 130° to about 180°C., have been found satisfactory. Dwell times in the heated zone will usually vary from about 2 seconds to about 1 minute, and more normally will be from about 3 to about 10 seconds. The important factor in selecting the heating conditions for optimum bonding is to heat the polyethylene sheath to at least its melting point. Thus, very high temperatures can be used with short exposure times, in order to achieve high speed operation. Specific conditions under which the thermal bonding is achieved are illustrated in more detail in the examples below.

In the thermal bonding step, the polyethylene surface of the conjugate fiber is fused so that where the fused surface touches another fiber, in particular the fused polyethylene surface of another conjugate fiber, welding or fusing together of the two fibers will occur. Upon cooling, the welds of fused polyethylene solidify, and excellent fiber-to-fiber bonds are thereby formed. Simple exposure to ambient air will ordinarily provide adequate cooling.

The thermal bonding step can be carried out by through-air bonding, as illustrated in Fig. 6 by the oven 60, or by other means such as infrared heating or other types of radiant heating. Through-air bonding is accomplished by carrying the web on a porous conveyer belt through a zone where hot-air is forced through the web. It can be carried through a heated zone between two porous screens or belts, or it can be carried around a rotating drum having a porous surface which is equipped to suck hot air through the web as it is passing around the drum. The exact method of effecting the heating has not been found to be narrowly critical.

After thermal bonding and cooling to solidify the bonds, the fabric 62 of the invention is collected, as on a conventional wind-up 64.

Certain of the fabrics of the invention have shown valuable utility as facing materials for absorbent products such as diapers and sanitary napkins wherein the nonwoven fabric of the invention is employed as the facing layer, with the absorbent layer in contact with the facing layer. The fabrics of the invention that are employed as facings generally have a rapid aqueous liquid penetration, that is a liquid penetration time of less than about 3 seconds, and they exhibit an extremely dry surface after aqueous liquid penetration. Their rewet is usually less than about 0.2 gram. (The liquid penetration and rewet test procedures are described below, in the Examples.)

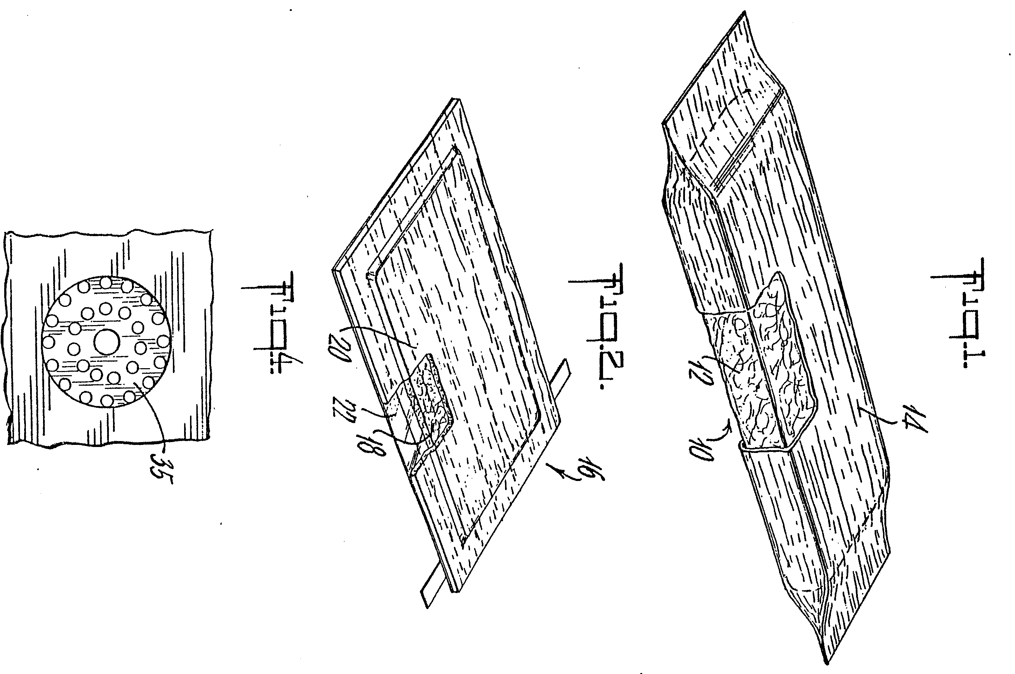

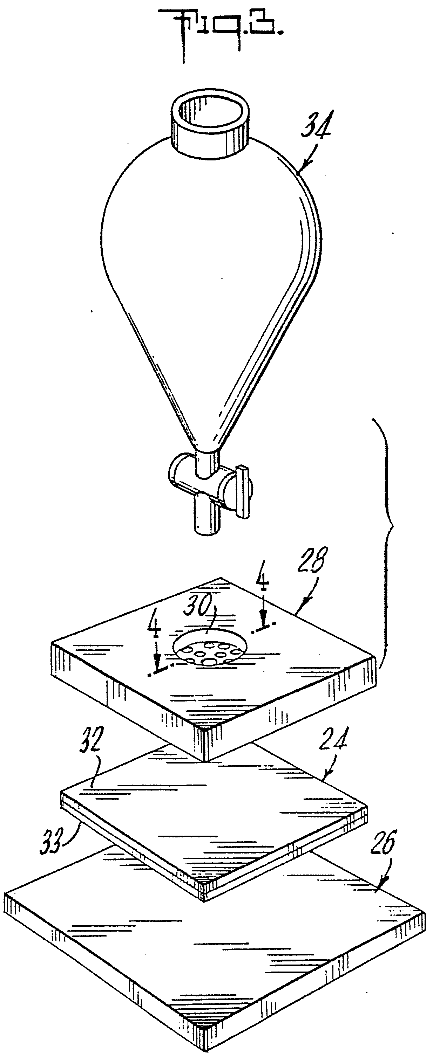

Figs. 1 and 2 illustrate a sanitary napkin and a disposable diaper, respectively, employing the nonwoven fabric of the invention as the facing. In Fig. 1, the sanitary napkin 10 comprises an absorbent core 12 wrapped in a liquid pervious cover sheet 14, which is the nonwoven fabric of the invention. In Fig. 2, the disposable diaper 16 comprises an absorbent core 18 contained between a liquid pervious facing 20, which is the nonwoven fabric of the invention, and a liquid impervious backing sheet 22.

In the Examples and Control Examples below, Fiber A and Fiber B were employed. Fiber A is a sheath/core conjugate fiber having a sheath of polyethylene having a density of about 0.96, a DSC melting point of about 132°C., and a melt index of about 42. The core is fiber grade polyethylene terephthalate. The weight ratio of the sheath and core is about 50/50. The fibers have a total denier of about 3, a tenacity of about 3.3 grams per denier, an elongation of 30 to 60 per cent, an average staple length of about 1.5 inches, and from 12 to 18 crimps per inch.

Fiber B, used for comparison purposes, is an eccentric sheath/core bicomponent fiber having a core of polypropylene and a sheath of high density polyethylene. The polyethylene had a softening range of 110°-120°C. and a melting point of about 130°C. The polypropylene had a softening range of 150°-160°C. and a melting point of about 165°C. The weight ratio of the sheath and core is about 50/50. The fibers have a staple length of about 1.5 inches, a total denier of about 3, a tenacity of 2.5-3.5 grams per denier, and from 10 to 13 crimps per inch.

Example 1 and Control Examples 1-2

Three different fabrics were produced by the following procedures:

Example 1 - 100 per cent Fiber A web laid by dual rotor, through-air bonded by passing around a drum having a porous surface adapted to suck hot air through the web. Exposed to hot air at a temperature of about 300°F. for about 7-8 seconds;

Control 1 - This is the facing fabric used on a commercial disposable diaper. It is a carded nonwoven composed of 65 weight per cent polyester fibers and 35 weight per cent acrylic binder. The fabric is embossed with a series of dots. The fabric is made following the teachings of U.S. Patent No. 4,041,951.

Control 2 - This is the facing fabric used on another commercial disposable diaper. This fabric is also made by following the teachings of U.S. Patent No. 4,041,951, but differs from Control 1 (a) in using a butyl acrylate/acrylamide copolymer binder, and (b) while the fabric is also embossed with a series of small dots, there are 5/8-inch diameter circular unembossed areas.

Selected physical properties of the fabrics of Example 1 and Controls 1 and 2 are displayed in Table I:

Table I

|  | Example 1 | Control 1 | Control 2 |
|---|---|---|---|
| Fiber Type | Fiber A | Polyester | Polyester |
| Weight, (OZ./SQ. YD.) | 0.8 | 0.7 | 0.7 |
| Thickness (MILS) | 39 | 8 | 10 |
| Wet MD Tensile (GMS/IN.) | 977 | 1773 | 1833 |
| Wet CD Tensile (GMS/IN.) | 612 | 166 | 214 |
| Liquid Penetration[1] (Seconds) | 1 | 4.8 | 49 |
| Rewet[2] (GMS) | 0.11 | 0.25 | 2.0 |

-----------------------------------------------------------------

(1) Liquid Penetration Time -

The purpose of this test is to measure the time required for saline solution to pass through the facing fabric of a disposable diaper. The fabric to be tested is first assembled into a disposable diaper with the test fabric as the facing, and then subjected to the following test procedure:

EQUIPMENT

A. 5 ml. pipet with a drain time between 6 and 10 seconds or automatic pipet.

B. Stop watch or timer.

C. 3.0 in. x 4.0 in. pressure plate of C.A. #316 bronze, with smooth, flat bottom surface and 3/4" smooth tapered hole in the center. The hole has a 3/4-inch diameter at the bottom, a 1.75-inch diameter hole at the top, and a taper of 25°, measured up from the horizontal. The taper begins 7/16-inch down from the top of the hole and ends 0.04 inch from the bottom of the hole. The total plate thickness is 1/2-inch. The plate shall weigh 300 ± 3 grams and shall show no signs of corrosion on the bottom or inside the tapered hole.

D. 1.59% dyed saline solution at room temperature. Prepared by dissolving 15.9 grams sodium chloride (USP grade or equivalent) and 0.5 grams Xylene Red B dye in demineralized water to make one liter total solution.

PROCEDURE

A. Place an opened diaper on a flat level surface with the fabric up. Place the pressure plate so that the hole will be on an area free from creases and excessive wrinkles.

Test must be made over the panel area.

B. Fill the pipet to the 5 ml. mark. Drain the solution onto the tapered surface of the hole, to avoid draining directly onto the diaper. (This is done to simulate a natural void as closely as possible.) If an automatic pipet is used, release the solution in 6 to 10 seconds.

C. Start the watch when the solution contacts the fabric. Stop the watch when there is no pool of liquid on any part of the fabric, even though the pipet is not completely drained. If the solution penetrates the fabric the instant it makes contact, the penetration time is termed "instantaneous."

REPORTING

A.   Test 10 diapers, one test per diaper.

B.   Record the penetration time to the nearest 0.1
second.

C.   If the liquid has not penetrated within one minute,
record the time as 60+.

D.   Report the average and range for the 10 tests.   In
calculating the average, assume a value of 60 for any 60+
figures.   Report 60+ for the range however.

(2)   The rewet test is carried out as follows:

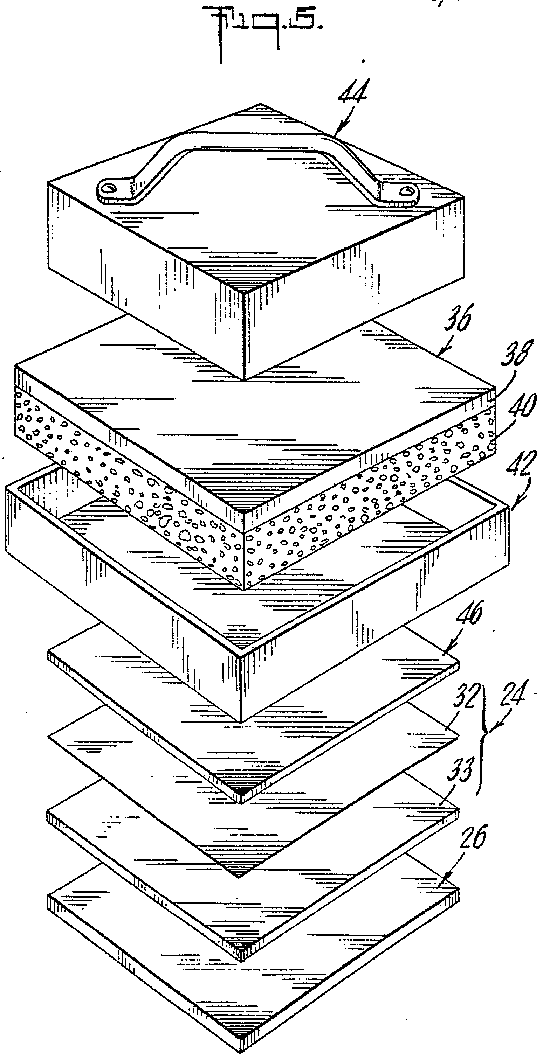

Figs. 3 and 4 show the apparatus used to carry out the
test.   Referring to Fig. 3, an absorbent pad 24 approximately four inches square is placed on a plastic base
plate 26 approximately five inches square.   The absorbent
pad 24 includes the facing fabric 32 to be tested for
liquid penetration, and an absorbent layer 33.   A strike-
through plate 28 is placed on top of the absorbent pad 24.
The strike-through plate 28 contains a cavity 30 in its
center.   The perforated plate at the base of the cavity 30
is depicted in Fig. 4 as number 35.   A separatory funnel
34 is placed so that the funnel tip is one inch above the
plastic plate 26.

An amount of 1% saline solution equal to the weight in
grams of the absorbent pad 24 times four is added to a
burette (not shown).   Five milliliters of this solution is
added to the funnel 34.   The five milliliters of solution
is discharged into the cavity 30.

Discharge the remaining saline solution from the burette
into the funnel 34 and then into the cavity 30.   Remove the burette, funnel 34, and strike-through plate 28. Referring now to Fig. 5, a pressure pad 36 is placed on top of the fabric 24. The pressure pad 36 consists of a square of plastic 38 affixed to a square of foam 40 covered by a polyethylene sheet 42. Lastly, a loading weight 44 is placed on the pressure pad 36. The total weight of the pressure pad 36 and the loading weight 44 is eight pounds.

The weights 36, 44 are left on the fabric sample 24 for three minutes. After the three minutes, the weights are removed and the pressure pad 36 is wiped dry. A weighed square of filter paper 46 is placed on top of the fabric 24. The pressure pad 36 and loading weight 44 are then placed on top. The sample is allowed to equilibrate for 2.0 minutes. The filter paper 46 is then removed and weighed to the nearest 0.001 gram. Rewet is equal to the difference in weight between the wet and dry filter paper, to the nearest 0.01 gram.

---

Example 2 and Control Example 3

Carded webs of both Fiber A (Example 2) and Fiber B (Control Example 3) were produced on an apparatus similar to that shown in Fig. 6, except that the webs were thermal bonded by passing them around a drum having a porous surface adapted to suck hot air through the web. The heating zone in the drum is 4.7 feet long. Tables II and III display the hot air temperatures, conveyor speeds, web weights, and certain other properties of the thermal bonded fabrics:

## Table II

### Example 2 Fabrics

| Temp. °C. | Speed Ft./min. | Weight grn./yd.[2] | Strip[1] Tensile lb./in | | Tenacity[2] | | Bulk, Mils | Density, gr/cm³ | Compressibility[3] Inches |
|---|---|---|---|---|---|---|---|---|---|
| | | | MD | CD | MD | CD | | | |
| 145 | 30 | 291 | 4.54 | 0.36 | 6.78 | 0.54 | 46 | 0.019 | 0.053 |
| 145 | 90 | 284 | 3.43 | 0.31 | 5.44 | 0.47 | 49 | 0.018 | 0.051 |
| 145 | 150 | 265 | 3.65 | 0.27 | 5.98 | 0.45 | 40 | 0.020 | 0.053 |
| 155 | 30 | 295 | 5.35 | 0.44 | 8.23 | 0.64 | 54 | 0.017 | 0.051 |
| 155 | 90 | 287 | 3.53 | 0.43 | 5.60 | 0.63 | 43 | 0.019 | 0.055 |
| 155 | 150 | 271 | 4.07 | 0.32 | 6.90 | 0.48 | 43 | 0.019 | 0.053 |
| 165 | 30 | 270 | 4.50 | 0.46 | 7.76 | 0.70 | 40 | 0.021 | 0.052 |
| 165 | 90 | 294 | 3.10 | 0.59 | 8.09 | 0.83 | 48.6 | 0.018 | 0.053 |
| 165 | 150 | 271 | 5.06 | 0.43 | 8.30 | 0.68 | 40.8 | 0.020 | 0.049 |

(1) Strip tensile strength is determined on an Instron Universal Testing Instrument using a 1 x 6-inch sample, with the crosshead speed set at 12 inches per minute, and the tension load set so that break occurs between 15% and 85% of full scale.

(2) Tenacity is calculated as strip tensile strength in pounds per inch divided by basis weight in ounces per square yard.

(3) Compressibility Test described below.

## Table III
### Control 3 Fabrics.

| Temp. °C. | Speed Ft./min. | Weight grn./yd. | Tensile, lb./in. | | Tenacity | | Bulk, Mils | Density, gr/cm | Compressibility Inches |
|---|---|---|---|---|---|---|---|---|---|
| | | | MD | CD | MD | CD | | | |
| 145 | 30 | 291 | 7.69 | 0.65 | 12.0 | 0.94 | 25.6 | 0.035 | 0.021 |
| 145 | 90 | 268 | 5.59 | 0.40 | 9.47 | 0.71 | 22.4 | 0.034 | 0.024 |
| 145 | 150 | 280 | 5.38 | 0.40 | 8.28 | 0.63 | 19.2 | 0.044 | 0.023 |
| 155 | 30 | 246 | 8.95 | 0.57 | 15.43 | 1.05 | 11.2 | 0.067 | 0.009 |
| 155 | 90 | 258 | 8.28 | 0.68 | 14.28 | 1.13 | 10.2 | 0.078 | 0.008 |
| 155 | 150 | 268 | 8.76 | 0.55 | 14.13 | 0.90 | 11.4 | 0.072 | 0.006 |
| 165 | 30 | 300 | 9.27 | 0.56 | 13.63 | 0.81 | 11.6 | 0.079 | 0.007 |
| 165 | 90 | 293 | 7.66 | 0.56 | 11.97 | 0.80 | 12.2 | 0.073 | 0.009 |
| 165 | 150 | 293 | 7.11 | 0.42 | 11.29 | 0.59 | 12.0 | 0.074 | 0.008 |

-14-

With carded webs of the same initial thickness and the same grain weights, the fabric of the invention maintains its low density, whereas the fabric made from Fiber B does not. The fabrics made from Fiber A have a soft, bulky, desirable feel to them, somewhat reminiscent of light weight cotton flannel. The fabrics of Fiber B have a harsher feel, and are considerably less bulky. This difference in feel of the fabrics shows up, at least in part, in a fabric compressibility test, which is carried out as follows:

## Compressibility Test

### Equipment Used

Refer to Fig. 11. The parts of the apparatus are as follows:

Top (load) indicator dial gauge 70
Bottom (thickness) indicator dial gauge 72
Pressure Foot 2" diameter 74
Top Load Balance, capacity 1,000 gms. (not shown)

### Calibration Procedure

1. Loosen the lock screws 76, 77 and swivel the instrument on the column 78 so that the presser foot 74 is aligned over the center of the flat surface of the top load balance.

Zero the top gauge dial 70, if necessary, by loosening the small knurled locking screw on the gauge and rotating the dial face to match the pointer at zero. Tighten locking screw.

Zero the balance.

Lower the presser foot 74 to the flat top surface of the balance.

Read weight on the balance as the load on the upper force gauge dial 70 is increased by turning the force knob 80.

| Reading on Top (force) Gauge 70 | Wt. - Grams On Balance | Force Calculated as lbs./sq. inch |
|---|---|---|
| 0.001 | 8.9 | 0.006 |
| 0.005 | 33.3 | 0.023 |
| 0.028 | 143.4 | 0.100 |
| 0.055 | 338.0 | 0.237 |
| 0.080 | 525.8 | 0.368 |
| 0.110 | 724.7 | 0.507 |

2. After the top "force" gauge 70 has been correlated to psi, swivel the instrument to realign the presser foot 74 over the base 82.

Lower the presser foot 74 until the foot 74 just makes contact with the base 82 (top gauge dial is still at zero).

Adjust the bottom gauge dial 72 to zero if necessary.

Testing

Place the preweighed test sample (not shown) on the base 82 under the foot 74 and lower the foot 74. Take first reading on the thickness gauge 72 as the top gauge dial 70 pointer moves off zero to 0.001. Continue readings at intervals up to 0.110 (equivalent to 0.5 psi).

Test several areas on each sample. If the desired reading on upper dial 70 is passed by, do not return to the lower reading but either continue on or start over in a new area.

Compressibility in inches = (thickness at 0.001 top gauge reading) minus (thickness at 0.110 top gauge reading)

The larger the difference between the thickness at 0.006 psi and 0.5 psi, the more compressible or softer the fabric.

---

Samples of Fibers A and B, and samples of the fabrics described above in Tables II and III that had been produced using a hot air temperature of 145°C. at a conveyor speed of 90 feet per minute, were measured for birefringence of the polyester or polypropylene cores. The procedure described by A.N.J. Heyn, "Fiber Microscopy", Interscience Publishers Inc., NY (1954), Chapter XXI, pages 321 et seq., and by Sieminski in "A Note on the Measurement of Birefringence in Fibers", pages 35-36, The Microscope, Volume 23 (1975), was employed. Each value shown in Table IV, below, is the average of five determinations.

Table IV

| Birefringence of Conjugate Fiber Cores | |
| --- | --- |
| Original Fiber A | 0.214 |
| Fiber A in Fabric | 0.214 |
| Original Fiber B | 0.046 |
| Fiber B in Fabric | 0.027 |

The significance of these measurements is that the thermal bonding treatment of Fiber A did not alter the birefringence of the polyester core, whereas the birefringence of the polypropylene core of Fiber B was reduced by about 41 per cent of its original value. Fiber B cannot be thermally bonded to form useful fabrics at bonding conditions that are significantly milder than those shown in Table III. Thus, at all conditions under which Fiber B can be thermally bonded to form a useful fabric, birefringence loss in the polypropylene core occurs. The loss in birefringence of the polypropylene in Fiber B is even greater under more extreme thermal bonding conditions (i.e., higher temperatures and/or longer exposure times), whereas more extreme thermal bonding conditions have not been found to significantly affect the birefringence of the polyester core of Fiber A. Since birefringence is a measure of orientation, the maintenance of the original orientation of the polyester core of Fiber A is believed to be related to the excellent combination of strength, low bulk density, and softness properties of the fabrics of the invention, when thermally bonded under a wide variety of conditions.

The literature reports that the birefringence of polyester fibers is normally at least about 0.16. Therefore, it is expected that the birefringence of the polyester in the thermal bonded nonwoven fabrics of the invention will be at least about 0.16.

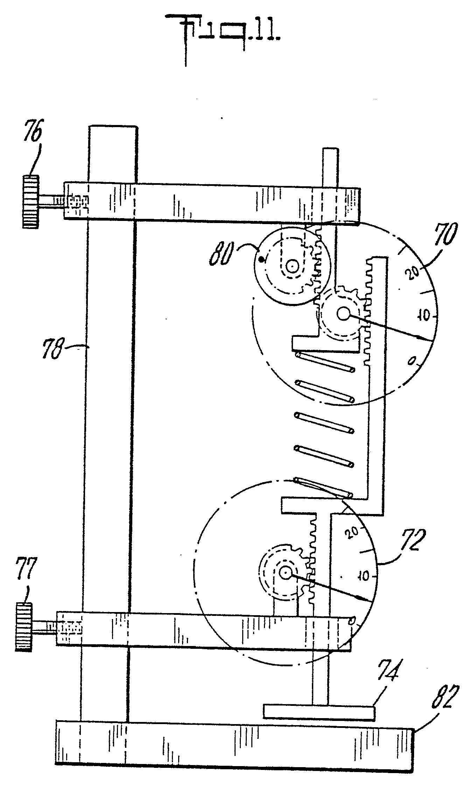

The fact that the polyester cores of the polyester/ polyethylene conjugate fibers maintain all, or almost all, of their original degree of orientation when thermally bonded to form the fabric of this invention, whereas the polypropylene cores of the polypropylene/polyethylene conjugate fibers do not, is demonstrated in Figs. 7 through 10. When the polyester/polyethylene conjugate fibers (Fig. 7) are thermally bonded to form a nonwoven fabric (Fig. 8 - the same fabric used for the birefringence measurements), there is little if any discernible change in the degree of curl of the individual fibers. However, when the polypropylene/polyethylene fibers (Fig. 9) are thermally bonded to form a nonwoven fabric (Fig. 10 - again, the fabric that is shown is the one that was used for birefringence measurements), the individual fibers straighten out to a considerable degree. This straightening out is evidence of the shrinkage that results when the polypropylene core loses some of its molecular orientation.

The foregoing Examples illustrate the production of fabrics of the invention having valuable utility as facing fabrics for absorbent products. In addition to their excellent aqueous liquid penetration and rewet properties, these fabrics are soft, strong, and bulky. Their bulk densities are usually within the range of from 0.01 to 0.15, preferably from 0.01 to 0.07, and more preferably from 0.015 to 0.04, grams per cubic centimeter, when used for facings.

There are a number of factors that combine to contribute to the desirable combination of strength, low bulk density, and softness of the fabrics of the invention. During the thermal bonding of the fibers, there is little, if any, shrinkage of the individual fibers. As a result, low bulk density and softness is maintained. The lack of shrinkage is related to the fact that the polyester portion of the fibers undergoes no significant change in orientation during the thermal bonding. The polyethylene, especially the relatively high melt index high density polyethylene, forms good bonds during the thermal bonding, even with no significant compression on the starting fibers to increase fiber-to-fiber contact.

CLAIMS:

1.    A thermal bonded nonwoven fabric having a bulk density of from 0.01 to 0.15 grams per cubic centimeter, and comprising thermal bonded polyester/polyethylene conjugate fibers, said fabric having an excellent combination of strength, low bulk density, and softness, wherein the birefringence of the polyester in the fibers of said fabric is not significantly different from that of the polyester in the fibers prior to thermal bonding.

2.    A thermal bonded nonwoven fabric having a bulk density of from 0.01 to 0.15 grams per cubic centimeter, and comprising thermal bonded polyester/polyethylene conjugate fibers, said fabric having an excellent combination of strength, low bulk density, and softness, wherein the birefringence of the polyester in the fibers of said fabric is at least about 0.16.

3.    The fabric of claim 1 or 2 wherein the polyester/polyethylene conjugate fibers have polyester cores and polyethylene sheaths.

4.    The fabric of any one of claims 1 to 3 wherein the polyethylene component of said conjugate fiber has a density of at least about 0.94 grams per cubic centimeter and a melt index of at least about 10.

5.    The fabric of any one of claims 1 to 4 wherein said fabric has a strikethrough time of not more than about 3 seconds, a rewet of less than about 0.1 gram, and a bulk density of from 0.01 to 0.07 grams per cubic centimeter.

6.    An absorbent product including a facing and an absorbent layer in contact within said facing, wherein said facing is the nonwoven fabric of any one of claims 1 to 5.

7.    The absorbent product of claim 6 in the form of a disposable diaper.

8.    The absorbent product of claim 6 in the form of a sanitary napkin.

Fig.1.

14

12

10

Fig.2.

16

20

22

18

Fig.4.

35

Fig.3.

Fig.5.

Fig. 6.

Fig.7.

Fig.8.

Fig.9.

Fig.10.

Fig.11.